# EUROPÄISCHE PATENTSCHRIFT

(11) **EP 1 952 831 B1**
(45) Veröffentlichungstag und Bekanntmachung des Hinweises auf die Patenterteilung: **12.05.2010**
(21) Anmeldenummer: 07024572.5
(22) Anmeldetag: 19.12.2007
(51) Int. Cl.: A61L 31/16

(54) **Mit Biomolekülen beschichtete Stents sowie Verfahren zu deren Herstellung**
Stent covered with biomolecules and method for its production
Stents revêtus de biomolécules et leur procédé de fabrication

(30) Priorität: 25.01.2007 DE 102007003708
(43) Veröffentlichungstag der Anmeldung: 06.08.2008
(73) Patentinhaber: Biotronik VI Patent AG, 6341 Baar (CH)
(72) Erfinder: Borck, Alexander Dr., 91086 Aurachtal (DE); Adden, Nina Dr., 38106 Braunschweig (DE)
(74) Vertreter: Lindner-Vogt, Karin L.

(56) Entgegenhaltungen:
- DE-A1- 10 325 049
- US-A1- 2003 158 460
- US-A1- 2004 265 571
- US-A1- 2006 135 476
- US-A1- 2006 194 008

## Beschreibung

Die vorliegende Erfindung betrifft einen Stent umfassend oder bestehend aus a) einem Stentgrundkörper, b) einer oder mehrerer Ankergruppen auf der Oberfläche des Stentgrundkörpers und c) einem oder mehreren Biomolekülen, die an das oder die Ankergruppen gebunden sind, ein Verfahren zur Herstellung eines erfindungsgemäßen Stents sowie die Verwendung von Verbindungen der allgemeinen Formel (I) (R²O)₂(O)P-L-(CH₂)ₓ-M-R¹ (wobei die Substituenten im Nachfolgenden beschrieben sind) als Ankergruppen für Biomoleküle aus Stentgrundkörpern sowie als Ankergruppen zur Herstellung als eines oder mehrerer erfindungsgemäßer Stents.

Stents im Allgemeinen sind endovaskuläre Prothesen beziehungsweise Implantate, die beispielsweise zur Behandlung von Stenosen verwendet werden. Stents sind außerdem bekannt für die Behandlung von Aneurismen. Stents weisen grundsätzlich eine Tragstruktur auf, die geeignet ist, die Wand eines Gefäßes in geeigneter Weise abzustützen, um so das Gefäß zu weiten beziehungsweise ein Aneurisma zu überbrücken. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischen Metall, wie Nitinol, aufgebaut.

Stents werden derzeit in zwei Grundtypen eingeteilt, die dauerhaften Stents und die biodegradierbaren Stents. Dauerhafte Stents sind so ausgestaltet, dass sie im Gefäß für einen unbestimmten Zeitraum verbleiben können. Biodegradierbare Stents hingegen werden über einen vorbestimmten Zeitraum hinweg in einem Gefäß abgebaut. Vorzugsweise werden biodegradierbare Stents erst abgebaut, wenn das traumatisierte Gewebe des Gefäßes verheilt ist und somit der Stent nicht weiter im Gefäßlumen verbleiben muss.

Das Dokument US2006194008 offenbart Implantate wie z.B. Gefäßersatzbauteile, Stents & Katheter beschichtet mit einer phosphorhaltigen Schicht wie z.B. 11-Hydroxyundecylphosphorsäure & Octadecylphosphorsäure, welche mit einem Protein (wie z.B. RGD) funktionalisiert werden können.

Das Dokument US2004265571 offenbart einen phosphanundecanol funktionalisierten Stent & ein Verfahren zu dessen Herstellung.

Es hat sich allerdings gezeigt, dass durch die Einbringung von Stents in Gefäßsysteme Nebenwirkungen, wie zum Beispiel Restenosen und Thrombosen auftreten können.

Die Aufgabe der vorliegenden Erfindung besteht darin, einen Stent bereitzustellen, der bei Einbringung in das Gefäßsystem gegenüber Stents des Standes der Technik ein vermindertes Restenoserisiko aufweist.

Diese Aufgabe wird erfindungsgemäß durch die Gegenstände der vorliegenden Patentansprüche gelöst. Bevorzugte Ausgestaltungen werden in den abhängigen Ansprüchen dargestellt.

Demzufolge löst ein Stent umfassend oder bestehend aus
a) einem Stentgrundkörper
b) einer oder mehreren Ankergruppen auf der Oberfläche des Stentgrundkörpers
c) einem oder mehreren Biomolekülen, die an das oder die Ankergruppen gebunden sind,
   dadurch gekennzeichnet, dass
   die Ankergruppen gleich oder verschieden ausgewählt werden aus der Gruppe der Verbindungen der allgemeinen Formel (I)

   (R²O)₂(O)P-L-(CH₂)ₓ-M-R¹ (I)

   wobei
   - R¹: für -COOH, -OH, -SH, -NH₂, Benzophenon oder Benzophenon-Derivate steht, bevorzugt für -COOH oder -NH₂,
   - R²: für Wasserstoff, -CH₂CH₃ oder -CH₃ steht, bevorzugt für Wasserstoff,
   - L: für eine Einfach-Bindung oder -O- steht, bevorzugt für eine Einfach-Bindung,
   - M: für -(CH₂-CH₂-O)_{y} steht,
   - x: für eine Zahl ausgewählt aus der Gruppe bestehend aus 1 bis 25 steht, bevorzugt für 3, 4, 5, 6, 7, 8, 9 oder 10 steht und
   - y: für eine Zahl ausgewählt aus der Gruppe bestehend aus 1 bis 25 steht, bevorzugt für 3, 4, 5, 6, 7, 8, 9 10, 11 oder 12 steht
   sowie deren Salze und Hydrate,
   und
   das oder die Biomoleküle gleich oder verschieden ausgewählt werden aus der Gruppe bestehend aus Verbindungen, welche die Anlagerung der endothelialen Vorläuferzellen an die Stentoberfläche unterstützen
die erfindungsgemäße Aufgabe.

Hierbei können die bevorzugten Ausgestaltungen des Stents alle zusammen, teilweise - in beliebiger Kombination - und einzeln vorhanden sein.

Ein weiterer Gegenstand der vorliegenden Erfindung betrifft ein Verfahren zum Herstellen eines erfindungsgemäßen Stents, dadurch gekennzeichnet, dass, das Verfahren umfasst, dass
a) ein oder mehrere Stentgrundkörper bereitgestellt werden,
b) der oder die Stentgrundkörper gereinigt werden,
c) eine oder mehrere, gleiche oder verschiedene Ankergruppen ausgewählt aus der Gruppe an Verbindungen der allgemeinen Formel (I)

   (R²O)₂(O)P-L-(CH₂)ₓ-M-R¹ (I)

   wobei
   - R¹: für -COOH, -OH, -SH, -NH₂, Benzophenon oder Benzophenon-Derivate steht, bevorzugt für -COOH oder -NH₂,
   - R²: für Wasserstoff, -CH₂CH₃ oder -CH₃ steht, bevorzugt für Wasserstoff,
   - L: für eine Einfach-Bindung oder -0- steht, bevorzugt für eine Einfach-Bindung,
   - M: für -(CH₂-CH₂-O)_{y} steht,
   - x: für eine Zahl ausgewählt aus der Gruppe bestehend aus 1 bis 25 steht, bevorzugt für 3, 4, 5, 6, 7, 8, 9 oder 10 steht und
   - y: für eine Zahl ausgewählt aus der Gruppe bestehend aus 1 bis 25 steht, bevorzugt für 3, 4, 5, 6, 7, 8, 9 10, 11 oder 12 steht
   sowie deren Salze und Hydrate bereitgestellt werden,
d) der oder die gereinigten Stentgrundkörper aus b) anschließend mit einer oder mehreren, gleichen oder verschiedenen Ankergruppen ausgewählt aus der Gruppe der Verbindungen der allgemeinen Formel (I) aus c) funktionalisiert werden,
e) ein oder mehrere, gleiche oder verschiedene Biomoleküle aus der Gruppe bestehend aus Verbindungen, welche die Anlagerung der endothelialen Vorläuferzellen an die Stentoberfläche unterstützen bereitgestellt werden
   und
f) ein oder mehrere, gleiche oder verschiedene Biomoleküle aus e) an den oder die funktionalisierten Stentgrundkörper aus d) gebunden werden.

Hierbei können die bevorzugten Ausgestaltungen der Ankergruppen alle zusammen, teilweise - in beliebiger Kombination - und einzeln vorhanden sein.

Ein weiterer Gegenstand der vorliegenden Erfindung besteht in der Verwendung von ein oder mehreren Verbindungen ausgewählt aus der Gruppe der allgemeinen Formel (I)

(R²O)₂(O)P-L-(CH₂)ₓ-M-R¹ (I)

wobei
- R¹: für -COOH, -OH, -SH, -NH₂, Benzophenon oder Benzophenon-Derivate steht, bevorzugt für -COOH oder -NH₂,
- R²: für Wasserstoff, -CH₂CH₃ oder -CH₃ steht, bevorzugt für Wasserstoff,
- L: für eine Einfach-Bindung oder -0- steht, bevorzugt für eine Einfach-Bindung,
- M: für -(CH₂-CH₂-O)_{y} steht,
- x: für eine Zahl ausgewählt aus der Gruppe bestehend aus 1 bis 25 steht, bevorzugt für 3, 4, 5, 6, 7, 8, 9 oder 10 steht und
- y: für eine Zahl ausgewählt aus der Gruppe bestehend aus 1 bis 25 steht, bevorzugt für 3, 4, 5, 6, 7, 8, 9 10, 11 oder 12 steht
sowie deren Salze und Hydrate
als Ankergruppen für Biomoleküle auf Stentgrundkörpern.

Hierbei können die bevorzugten Ausgestaltungen der Ankergruppen alle zusammen, teilweise - in beliebiger Kombination - und einzeln vorhanden sein.

Zudem besteht ein weiterer Gegenstand der vorliegenden Erfindung in der Verwendung von Verbindungen der allgemeinen Formel (I)

(R²O)₂(O)P-L-(CH₂)ₓ-M-R¹ (I)

wobei
- R¹: für -COOH, -OH, -SH, -NH₂, Benzophenon oder Benzophenon-Derivate steht, bevorzugt für -COOH oder -NH₂,
- R²: für Wasserstoff, -CH₂CH₃ oder -CH₃ steht, bevorzugt für Wasserstoff,
- L: für eine Einfach-Bindung oder -0- steht, bevorzugt für eine Einfach-Bindung,
- M: für -(CH₂-CH₂-O)_{y} steht,
- x: für eine Zahl ausgewählt aus der Gruppe bestehend aus 1 bis 25 steht, bevorzugt für 3, 4, 5, 6, 7, 8, 9 oder 10 steht und
- y: für eine Zahl ausgewählt aus der Gruppe bestehend aus 1 bis 25 steht, bevorzugt für 3, 4, 5, 6, 7, 8, 9 10, 11 oder 12 steht
sowie deren Salze und Hydrate,
als Ankergruppen zur Herstellung eines oder mehrerer erfindungsgemäßer Stents.

Hierbei können die bevorzugten Ausgestaltungen der Ankergruppen alle zusammen, teilweise - in beliebiger Kombination - und einzeln vorhanden sein.

Der vorliegenden Erfindung liegt die Erkenntnis zugrunde, dass durch die erfindungsgemäße Beschichtung der Stents mit Biomolekülen endotheliale Vorläuferzellen (Endothelial Progenitor Cells, EPCs) an der Stentoberfläche gebunden werden. Dies führt dazu, dass die erfindungsgemäße Stentoberfläche im Vergleich zu einem Stent des allgemeinen Stand der Technik schneller mit Endothelialzellen (Endothelial Cells, EC) bewachsen werden. Folglich wird die Endothelialschicht des Gefäßes im Bereich der Stentapplikation schneller aufgebaut und so das Restenoserisiko vermindert. Das schnelle und komplette Überwachsen mit Endothel verhindert Spätthrombosen, wie sie insbesondere bei wirkstofffreisetzenden Stents beobachtet werden können. Den aus dem Stand der Technik bekannten wirkstofffreisetzenden Stents ist gemeinsam, dass sie Polymere und/ oder Wirkstoffe verwenden, die die Endothelialisierung behindern, und so zu den geschriebenen Spätthrombosen führen.

Überraschenderweise wurde ebenfalls erkannt, dass durch die Verwendung von Verbindungen der allgemeinen Formel (I), wobei die Substituenten oben beschrieben wurden, als Ankergruppen auf den Stentgrundkörpern das Entzündungsrisiko und/oder das Thromboserisiko im Vergleich zu Polymeren vermindert wird. Es wird angenommen, dass Entzündungen und/oder Thrombosen durch den Abbau der Polymere, z. B. der Polyurethane oder einiger Polyester, hervorgerufen werden. Im Gegensatz hierzu werden die erfindungsgemäßen Ankerverbindungen ausgewählt aus der Gruppe der Verbindungen der allgemeinen Formel (I) unter physiologischen Bedingungen nicht entzündungs- und/oder thrombosefördernd abgebaut.

Durch den erfindungsgemäßen Stent können zudem natürliche Mechanismen des Körpers genutzt werden, um die geschädigten Endothelschichten der Gefäße, bevorzugt im Bereich der Stentapplikation, wiederherzustellen. Hierunter sind u. a. die folgenden körpereigenen Mechanismen zu rechnen: Erzeugung von schnellerem und zielgerichteterem Wachstum von Endothel. Durch die schon frühzeitig geschlossene Zellschicht werden weniger Signalmoleküle wie Entzündungsmediatoren freigesetzt.

Unter einem Stentgrundkörper gemäß der vorliegenden Erfindung ist ein dauerhafter oder degradierbarer Metallstent oder ein Polymerstent zu verstehen.

### Permanenter Metallstent

Der Grundkörper des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung. In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol. In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl. Ferner kann der Grundkörper des Stents mindestens teilweise aus Kunststoff und/oder einer Keramik bestehen.

### Degradierbarer Metallstent

Vorzugsweise ist der biokorrodierbare metallische Werkstoff eine biokorrodierbare Legierung, ausgewählt aus der Gruppe Magnesium, Eisen und Wolfram; insbesondere ist der biokorrodierbare metallische Werkstoff eine Magnesiumlegierung.

Die Legierungen der Elemente Magnesium, Eisen oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert.

Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 0,0 - 5,5 Gew.%, und Rest < 1 Gew.% eingesetzt, wobei Magnesium den auf 100 Gew.% fehlenden Anteil an der Legierung einnimmt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

### Dauerhafter Polymerstent

Stentgrundkörper aus dauerhaften Polymerstents bestehen vorzugsweise aus Polypropylen, Polyethylen, Polyvinylchlorid, Polymethylmethylethylacrylat, Polymethylethylacrylat, Polytetrafluorethylen, Polyvinylalkohol, Polyurethan, Polybuthylenterephthalat, Silikonen, Polyphosphaten sowie deren Copolymere und Blends, oder Polyhydroxybuttersäure (ataktisch, isotaktisch, syndiotaktisch sowie deren Blends) etc.

### Degradierbarer Polymerstent

Stentgrundkörper aus degradierbaren Polymerstents bestehen vorzugsweise aus Polydioxanon, Polyglycolid Polycaprolacton, Polylactide [Poly-L-Lactid, Poly-D,L-Lactid, und Copolymere sowie Blends, wie Poly(L-Lactid-co-glycolid), Poly(D,L-lactid-co-glycolid), Poly(L-Lactid-co-D,L-Lactid), Poly(I-Lactid-co-trimethylen carbonat)], Triblockcopolymere, Polysaccharide [Chitosan, Levan, Hyaluronsäure, Heparin, Dextran, Cellulose etc.], Polyhydroxyvalerat, Ethylvinylacetat, Polyethylenoxid, Polyphosphorylcholin, Fibrin, Albumin etc.

Biodegradierbare und dauerhafte Metallstents sind gegenüber Polymerstents bevorzugt.

Gemäß der vorliegenden Erfindung werden ein oder mehrere Ankergruppen für einen erfindungsgemäßen Stent gleich oder verschieden ausgewählt aus der Gruppe der Verbindungen der allgemeinen Formel (I)
- R¹: für -COOH, -OH, -SH, -NH₂, Benzophenon oder Benzophenon-Derivate steht, bevorzugt für -COOH oder -NH₂,
- R²: für Wasserstoff, -CH₂CH₃ oder -CH₃ steht, bevorzugt für Wasserstoff,
- L: für eine Einfach-Bindung oder -O- steht, bevorzugt für eine Einfach-Bindung,
- M: für -(CH₂-CH₂-O)y steht,
- x: für eine Zahl ausgewählt aus der Gruppe bestehend aus 1 bis 25 steht, bevorzugt für 3, 4, 5, 6, 7, 8, 9 oder 10 steht und
- y: für eine Zahl ausgewählt aus der Gruppe bestehend aus 1 bis 25 steht, bevorzugt für 3, 4, 5, 6, 7, 8, 9 10, 11 oder 12 steht
sowie deren Salze und Hydrate.

Demzufolge fallen unter Verbindungen der allgemeinen Formel (I) Phosphonsäuren der allgemeinen Formel (II)

(HO)₂(O)P-O(CH₂)ₓ-M-R¹ (II)

und
deren Ester der allgemeinen Formel (III)

(R²O₂(O)P-CH₂)ₓM-R¹ (III)

sowie Verbindungen der phosphorigen Säure der allgemeinen Formel (IV)

(HO)₂(O)P-O-(CH₂)ₓM-R¹ (IV)

und/oder deren Ester der allgemeinen Formel (V)

(R²O)₂(O)P-O(CH₂)ₓ-M-R¹ (V),

wobei die (bevorzugten) Reste R¹, R², M, und x gemäß der allgemeinen Formel (I) definiert sind und die bevorzugten Reste alle zusammen, teilweise - in beliebiger Kombination - und einzeln vorhanden sein können.

Bei Verbindungen der allgemeinen Formeln (I), (II), (III), (IV) und (V), steht M für einen Oligoetylenglykol-Linker -((CH₂)CH₂O)_{Y}-. Hierdurch wird eine unspezifische Proteinanlagerung an diese Ankergruppen im Gegensatz zu Ankergruppen, in denen M für eine Einfach-Bindung steht, vermindert.

Unter Biomolekülen gemäß der vorliegenden Erfindung sind solche Verbindungen zu verstehen, welche die Anlagerung von endothelialen Vorläuferzellen an die Stentoberfläche fördern und so an der Endothelialisierung des erfindungsgemäßen Stents mitwirken, was zudem in Gefäßen, bevorzugt im Bereich der Stentapplikation, das aufgrund der Schädigung der Gefäße hervorgerufene vermehrte Wachstum von glatten Muskelzellen vermindert.

Als Biomoleküle kommen bevorzugt Proteine, wie zum Beispiel Protein A, Enzyme, Wachstumsfaktoren, Antikörper, wie zum Beispiel CD 133, aber auch Peptidsequenzen, wie zum Beispiel cyclic-RGD, in Frage. Bevorzugt ist der Antikörper CD133 und das Protein A, da diese eine gute Ausgangsbasis bieten, um Antikörper ausgerichtet zu binden.

Unter Bereitstellung eines Stentgrundkörpers gemäß der vorliegenden Erfindung ist die Bereitstellung eines nicht derivatisierten und/oder funktionalisierten Stents zu verstehen. Solche Stentgrundkörper bestehen aus den voranstehenden Stentmaterialien und besitzen eine Geometrie, die im Stand der Technik allgemein bekannt ist.

Unter dem erfindungsgemäßen Reinigungsschritt von Stentgrundkörpern ist eine Behandlung des Stentgrundkörpers zu verstehen, die den Stentgrundkörper so aktiviert, dass im anschließenden Schritt die Ankermoleküle an den Stentgrundkörper gebunden werden. Bevorzugt ist unter dem erfindungsgemäßen Reinigungsschritt die Reinigung in einem Sauerstoffplasma oder durch Spülen mit Lösungsmittel, bevorzugt der Lösungsmittelreihe Dichlormetan, Aceton, Methanol und Millipore Wasser, zu verstehen. Gegebenenfalls kann an den Reinigungsschritt ein Trocknungsschritt anschließen.

Unter Bereitstellung von Ankergruppen ausgewählt aus Verbindungen der allgemeinen Formel (I) ist eine Bereitstellung dieser Verbindungen gelöst, suspendiert oder emulgiert in einem Lösungsmittel, ausgewählt aus der Gruppe bestehend aus Methanol, Ethanol, Aceton, Tetrahydrofuran, Dimethylformamid, Chloroform, Dimethylsulfoxid, Dichlormethan etc., bevorzugt trockenes Tetrahydrofuran, zu verstehen.

Unter dem erfindungsgemäßen Funktionalisieren der gereinigten Stentgrundkörper mit einer oder mehreren, gleichen oder verschiedenen Ankergruppen ist das ganze oder teilweise In-Kontakt-Bringen des gereinigten Stentgrundkörper mit einer oder mehreren Lösungen gleicher und/oder verschiedener Ankergruppen der Verbindungen der Formel (I) zu verstehen, wobei das oder die Lösungsmittel anschließend ganz oder teilweise abgedampft werden, insbesondere wobei das oder die Lösungsmittel innerhalb von einer Stunde so abgedampft werden, dass der Meniskus der Lösung über die Stentoberfläche wandert. Unter In-Kontakt-Bringen ist bevorzugt ein Besprühen des gereinigten Stentgrundkörpers mit einer oder mehreren Lösungen, gleicher und/oder unterschiedlicher Ankergruppen oder ein Eintauchen des gereinigten Stentgrundkörpers in eine oder mehrere Lösungen, gleicher und/oder verschiedener Ankergruppen zu verstehen.

Anschließend kann hieran gegebenenfalls der mit einer oder mehreren Ankergruppen funktionalisierte Stent thermisch behandelt werden (getempert werden), bevorzugt über einen Zeitraum von 1 bis 124 Stunden, bevorzugt in einem Temperaturbereich von 60 bis 220°C. Bevorzugt wird für 18 bis 74 Stunden bei 100 bis 140°C getempert.

Gegebenenfalls wird der funktionalisierte Stent, thermisch behandelt oder nicht, anschließend mit Lösungsmittel gespült.

Anschließend kann der so vorbehandelte funktionalisierte Stent gegebenenfalls über einen Zeitraum von 1 bis 24 Stunden in eine Carbonyldiimidazol - Lösung (CDI) in trockenem Dioxan gelegt werden, bevorzugt über 15 Stunden in eine 0,3 M Lösung von Carbonyldiimidazol in trockenem Dioxan. Gegebenenfalls schließt hieran eine Spülung des funktionalisierten Stents mit trockenem Dioxan an.

Anschließend kann gegebenenfalls ein Trockenschritt im Stickstoffstrom folgen.

Unter Bereitstellung erfindungsgemäßer Biomoleküle ist eine Lösung, Suspension sowie Emulsion der Biomoleküle in einer Puffer-Lösung, die aminosäurefrei ist, zu verstehen. Als Puffer kommen Phosphat-Puffer, PBS-Puffer (phosphate buffered saline), MES-Puffer (2-Morpholinoethanesulfonic acid), Borat-Puffer etc. in Frage. Bevorzugt ist der PBS-Puffer.

Der oder die funktionalisierten Stents werden ganz oder teilweise mit der Lösung, Suspension oder Emulsion des Biomoleküls in dem Puffer in Kontakt gebracht und gegebenenfalls hiermit gespült. Unter In-Kontakt-Bringen ist bevorzugt ein Besprühen des funktionalisierten Stentgrundkörpers mit einer oder mehreren Puffer-Lösungen, gleicher und/oder verschiedener Biomoleküle oder ein Eintauchen des gereinigten Stentgrundkörpers in eine oder mehrere Puffer-Lösungen, gleicher und/oder verschiedener Biomoleküle zu verstehen.

Anschließend kann gegebenenfalls durch Trockenblasen mit Stickstoff ein Trocknungsschritt folgen.

Zur Ausbildung der Bindung der Biomoleküle an Benzophenon-Ankergruppen folgt ein Belichtungsschritt, zum Beispiel bei 260 nm mit 100 mW/cm².

Zur Ausbildung der Bindung der Biomoleküle an Carbonyl-Ankergruppen folgt eine Aktivierung mit NHS/EDC und daran folgende Kopplung des Biomoleküls.

### Beispiel 1: (kein Bestandteil der beanspruchten Erfindung)

Ein im Sauerstoffplasma oder durch Spülen mit der Lösungsmittelreihe Dichlormethan, Aceton, Methanol und Millipore Wasser gereinigter Stent wird im Folgenden weiterbehandelt:

Es wird eine 1 mM Lösung von Hydroxyundecylphosphonsäure in trockenem Tetrahydrofuran hergestellt. In diese wird der Stent gehängt und das Lösungsmittel wird innerhalb von einer Stunde abgedampft, wobei der Meniskus der Lösung über die Stentoberfläche wandert.

Anschließend wird der Stent 18 Stunden bei 120°C getempert und daraufhin mit Lösungsmittel gespült.

Der so vorbehandelte Stent wird 15 Stunden in eine 0,3 M Lösung von Carbonyldiimidazol (CDI) in trockenem Dioxan gelegt. Anschließend wird der Stent zweimal 10 Minuten mit trockenem Dioxan gespült und anschließend im Stickstoffstrom getrocknet.

Auf die so behandelten Stents wird eine Lösung von CD 133 (ca. 50 µg/ml) in PBS-Puffer (aminosäurefrei) gegeben und über Nacht bei 4°C geschüttelt. Anschließend wurden die Stents mit Puffer gespült.

Der Nachweis von gebundenem Protein erfolgt mit fluoreszensmarkierten Antigen zu CD133.

### Beispiel 2: (kein Bestandteil der beanspruchten Erfindung)

Ein gemäß Beispiel 1 gereinigter Stent wird im Folgenden weiterbehandelt:

Es wird eine 3 mM Lösung von 3-(4-Oxybenzophenone)propylphosphonsäure in trockenem Tetrahydrofuran hergestellt.

Mit dieser Lösung wird der gereinigte Stent dreimal besprüht. Anschließend wird der Stent 12 Stunden bei 120°C getempert und daraufhin mit Lösungsmittel gespült.

Diese Stents werden in eine Lösung CD133 (ca.500 µg/ml) in Puffer gegeben und über Nacht bei 4°C geschüttelt.

Am nächsten Tag werden die Stents aus dem Lösungsmittel entfernt, getrocknet und bei 260 nm mit 100 mW/cm² belichtet.

Nicht gebundenes Protein wird abgewaschen.

Der Nachweis von gebundenen Proteinen erfolgt mittels fluoreszensmarkierten Antigen zu CD133.

### Beispiel 3: (kein Bestandteil der beanspruchten Verbindung)

Ein gemäß Beispiel 1 gereinigter Stent wird im Folgenden weiterbehandelt:

Es wird eine 1 mM von Carboxydodecylphosphonsäure in trockenem Tetrahydrofuran hergestellt.

In diese wird der Stent gehängt und das Lösungsmittel wird innerhalb von einer Stunde abgedampft, wobei der Meniskus der Lösung über die Stentoberfläche wandert. Anschließend wird der Stent 74 Stunden bei 120° getempert und daraufhin mit Lösungsmittel gespült.

Der so vorbehandelte Stent wird 45 Minuten in einer 1:1 Mischung von 0,4 M EDC und 0,1 M NHS in Millipore Wasser gelegt. Anschließend wird er kurz mit Millipore Wasser gespült und im Stickstoffstrom getrocknet.

Auf diese Stents wird eine Lösung von CD133 (50 µg/ml) in Puffer (aminosäurefrei) gegeben und über Nacht bei 4°C geschüttelt.

Die Stents wurden mit Puffer gespült, um nicht gebundenes Protein abzuwaschen.

Der Nachweis von gebundenem Protein erfolgte mittels fluoreszensmarkiertem Antigen zu CD133.

## Patentansprüche

1. Stent umfassend oder bestehend aus
a) einem Stentgrundkörper,
b) einer oder mehreren Ankergruppen auf der Oberfläche des Stentgrundkörpers,
c) einem oder mehreren Biomolekülen, die an das oder die Ankergruppen gebunden sind,
**dadurch gekennzeichnet, dass**
die Ankergruppen gleich oder verschieden ausgewählt werden aus der Gruppe der Verbindungen der allgemeinen Formel (I)
(R²O)₂(O)P-L-(CH₂)ₓ-M-R¹ (I)
wobei
R¹ für -COOH, -OH, -SH, -NH₂, Benzophenon oder Benzophenon-Derivate steht,
R² für Wasserstoff, -CH₂CH₃ oder -CH₃ steht,
L für eine Einfach-Bindung oder -O- steht,
M für -(CH₂-CH₂-O)_{y} steht,
x für eine Zahl ausgewählt aus der Gruppe bestehend aus 1 bis 25 steht und
y für eine Zahl ausgewählt aus der Gruppe bestehend aus 1 bis 25 steht.

2. Stent gemäß Anspruch 1, **dadurch gekennzeichnet, dass**
das oder die Biomoleküle gleich oder verschieden ausgewählt werden aus der Gruppe bestehend aus Proteinen, vorzugsweise aus Protein A, Enzymen, Wachstumsfaktoren, Antikörpern, vorzugsweise Antikörper CD 133, und Peptidsequenzen, vorzugsweise cyclic-RGD.

3. Stent gemäß Anspruch 1 oder 2, **dadurch gekennzeichnet, dass**
der Stentgrundkörper ausgewählt wird aus permanenten oder degradierbaren Metallen oder degradierbaren Polymermaterialien.

4. Verfahren zum Herstellen eines mit Biomolekülen beschichteten Stents gemäß einem der Ansprüche 1 bis 3, **dadurch gekennzeichnet, dass** das Verfahren umfasst, dass
a) ein oder mehrere Stentgrundkörper bereitgestellt werden,
b) der oder die Stentgrundkörper gereinigt werden,
c) eine oder mehrere, gleiche oder verschiedene Ankergruppen bereitgestellt werden ausgewählt aus der Gruppe an Verbindungen der allgemeinen Formel (I)
(R²O)₂(O)P-L-(CH₂)ₖM-R¹ (I)
wobei
R¹ für -COOH, -OH, -SH, -NH₂, Benzophenon oder Benzophenon-Derivate steht,
R² für Wasserstoff, -CH₂CH₃ oder -CH₃ steht,
L für eine Einfach-Bindung oder -O- steht,
M für -(CH₂-CH₂-O)_{y} steht,
x für eine Zahl ausgewählt aus der Gruppe bestehend aus 1 bis 25 und
y für eine Zahl ausgewählt aus der Gruppe bestehend aus 1 bis 25 steht,
d) der oder die gereinigten Stentgrundkörper aus b) anschließend mit einer oder mehreren, gleichen oder verschiedenen Ankergruppen ausgewählt aus der Gruppe der Verbindungen der allgemeinen Formel (I) aus c) funktionalisiert werden,
e) ein oder mehrere, gleiche oder verschiedene Biomoleküle aus der Gruppe bestehend aus Verbindungen, welche die Anlagerung der endothelialen Vorläuferzellen an die Stentoberfläche fördern, bereitgestellt werden
und
f) ein oder mehrere, gleiche oder verschiedene Biomoleküle aus e) an den oder die funktionalisierten Stentgrundkörper aus d) gebunden werden.

5. Verfahren gemäß Anspruch 4, **dadurch gekennzeichnet, dass**
g) der oder die Stents gemäß Schritt b) in einem Sauerstoffplasma und/oder durch Spülen mit Lösungsmittel, bevorzugt der Lösungsmittelreihe Dichlormethan, Aceton, Methanol und Millipore Wasser, gereinigt und
h) gegebenenfalls getrocknet werden.

6. Verfahren gemäß Anspruch 4 oder 5, **dadurch gekennzeichnet, dass**
i) eine oder mehrere Ankergruppen ausgewählt aus Verbindungen der Formel (I) gemäß Schritt c) in einem oder mehreren Lösungsmitteln, ausgewählt aus der Gruppe bestehend aus Methanol, Ethanol, Aceton, Tetrahydrofuran, Dimethylfonuamid, Chloroform, Dimethylsulfoxid und Dichlormethan insbesondere
j) der oder die gereinigten Stents gemäß Schritt b) mit der Lösung der Ankergruppen der Verbindungen der Formel (I) ganz oder teilweise in Kontakt gebracht werden,
k) gegebenenfalls das oder die Lösungsmittel ganz oder teilweise abgedampft werden,
I) gegebenenfalls der oder die Stents mit einer oder mehreren Ankergruppen thermisch behandelt werden,
m) gegebenenfalls anschließend mit einem oder mehreren Lösungsmitteln gespült werden und
n) anschließend gegebenenfalls getrocknet werden.

7. Verfahren gemäß einem der Ansprüche 4 bis 6, **dadurch gekennzeichnet, dass**
o) das oder die Biomoleküle gemäß Schritt e) in einem oder mehreren Puffern, die aminosäurefrei sind, bereitgestellt werden,
p) der oder die funktionalisierten Stents mit dem oder den gleichen oder verschiedenen in Lösung gebrachten Biomolekülen ganz oder teilweise in Kontakt gebracht werden,
q) gegebenenfalls anschließend gespült werden und
r) gegebenenfalls anschließend getrocknet werden:

8. Verwendung von Verbindungen der allgemeinen Formel (I)
(R²OM)₂(O)P-L-(CH₂)ₓM-R¹ (I)
wobei
R¹ für -COOH, -OH, -SH, -NH₂, Benzophenon oder Benzophenon-Derivate steht,
R² für Wasserstoff, -CH₂CH₃ oder -CH₃ steht,
L für eine Einfach-Bindung oder -O- steht,
M für -(CH₂-CH₂-O)_{y} steht,
x für eine Zahl ausgewählt aus der Gruppe bestehend aus 1 bis 25 und
y für eine Zahl ausgewählt aus der Gruppe bestehend aus 1 bis 25 steht
als Ankergruppen für Biomoleküle auf Stentgrundkörpem.

9. Verwendung von Verbindungen der allgemeinen Formel (I)
(R²O)₂(O)P-L-(CH₂)ₓ-M-R¹ (I)
wobei
R¹ für -COOH, -OH, -SH, -NH₂, Benzophenon oder Benzophenon-Derivate steht,
R² für Wasserstoff, -CH₂CH₃ oder -CH₃ steht,
L für eine Einfach-Bindung oder -O- steht,
M für -(CH₂-CH₂-O)_{y} steht,
x für eine Zahl ausgewählt aus der Gruppe bestehend aus 1 bis 25 und
y für eine Zahl ausgewählt aus der Gruppe bestehend aus 1 bis 25 steht
als Ankergruppen zur Herstellung eines oder mehrerer Stents gemäß einem der Ansprüche 1 bis 3.

## Claims

1. A stent comprising or consisting of
a) a stent base body,
b) one or more anchor groups on the surface of the stent base body,
c) one or more biomolecules bound to the anchor group(s),
**characterized in that**
the anchor groups, which may be the same or different, are selected from the group of compounds of general formula (I)
(R²O)₂(O)P-L-(CH₂)ₓ-M-R¹ (I)
wherein
R¹ stands for COOH, OH, SH, NH_{2,} benzophenone or benzophenone derivatives,
R² stands for hydrogen, CH₂CH₃ or CH₃,
L stands for a single bond or -O-,
M stands for (CH₂-CH₂-O)_{y},
x stands for a number selected from the group consisting of 1 to 25, and
y stands for a number selected from the group consisting of 1 to 25.

2. The stent according to claim 1, **characterized in that** the biomolecule(s) is (are) the same or different and is (are) selected from the group consisting of proteins, preferably protein A, enzymes, growth factors, antibodies, preferably antibody CD 133, and peptide sequences, preferably cyclic RGD.

3. The stent according to claim 1 or 2, **characterized in that** the stent base body is selected from permanent or degradable metals or degradable polymer materials.

4. The method for manufacturing a stent coated with biomolecules according to any one of claims 1 to 3, **characterized in that** the method comprises
a) providing one or more stent base bodies,
b) purifying the stent base body (bodies),
c) providing one or more anchor groups, which may be the same or different and are selected from the group of compounds of general formula (I)
(R²O)₂(O)P-L-(CH₂)ₓ-M-R¹ (I)
wherein
R¹ stands for COOH, OH, SH, NH₂, benzophenone or benzophenone derivatives,
R² stands for hydrogen, CH₂CH₃ or CH₃,
L stands for a single bond or -O-,
M stands for (CH₂-CH₂-O)_{y},
x stands for a number selected from the group consisting of 1 to 25, and
y stands for a number selected from the group consisting of 1 to 25,
d) the cleaned stent base body (bodies) from b) (are) then functionalized with one or more anchor groups, which may be the same or different and are selected from the group of compounds of general formula (I) from c),
e) one or more biomolecules, which may be the same or different from the group consisting of compounds, which facilitate the addition of endothelial progenitor cells onto the stent surface, are provided,
and
f) one or more biomolecules, which may be the same or different from e), are bound to the functionalized stent base body (bodies) from d).

5. The method according to claim 4, **characterized in that**
g) the stent(s) according to step b) is (are) cleaned in an oxygen plasma and/or by rinsing with solvent, preferably of the solvent series including dichloromethane, acetone, methanol and Millipore water, and
h) drying, if necessary.

6. The method according to claim 4 or 5, **characterized in that**
i) one or more anchor groups selected from compounds of formula (I) according to step c) are provided in one ore more solvents selected from the group consisting of methanol, ethanol, acetone, tetrahydrofuran, dimethylformamide, chloroform, dimethyl sulfoxide and dichloromethane in particular,
j) the cleaned stent(s) according to step b) is (are) brought entirely or partially contact with the solution of anchor groups of the compounds of formula (I),
k) the solvent(s) is (are) entirely or partially evaporated, if necessary,
l) the stent(s) is (are) thermally treated with one or more anchor groups, if necessary.
m) then rinsed with one or more solvents, if necessary, and
n) next dried, if necessary.

7. The method according to any one of claims 4 to 6, **characterized in that**
o) the biomolecule(s) according to step e) is (are) provided in one or more buffers, which are free of amino acid,
p) the functionalized stent(s) is (are) brought entirely or partially in contact with the same or different biomolecule(s) in solution,
q) then rinsed, if necessary, and
r) then dried, if necessary.

8. The use of compounds of general formula (I)
(R²O)₂(O)P-L-(CH₂)ₓ-M-R¹ (I)
wherein
R¹ stands for COOH, OH, SH, NH₂, benzophenone or benzophenone derivatives,
R² stands for hydrogen, CH₂CH₃ or CH₃,
L stands for a single bond or -O-,
M stands for (CH₂-CH₂-O)_{y},
x stands for a number selected from the group consisting of 1 to 25, and
y stands for a number selected from the group consisting of 1 to 25 as anchor groups for biomolecules on stent base bodies.

9. The use of compounds of general formula (I)
(R²O)₂(O)P-L-(CH₂)ₓ-M-R¹ (I)
wherein
R¹ stands for COOH, OH, SH, NH₂, benzophenone or benzophenone derivatives,
R² stands for hydrogen, CH₂CH₃ or CH₃,
L stands for a single bond or -O-,
M stands for (CH₂-CH₂-O)_{y},
x stands for a number selected from the group consisting of 1 to 25, and
y stands for a number selected from the group consisting of 1 to 25, as anchor groups for production of one or more stents according to one or more claims 1 to 3.

## Revendications

1. Stent, comprenant ou consistant en :
a) un corps de base de stent,
b) un ou plusieurs groupes d'ancrage sur la surface du corps de base de stent,
c) une ou plusieurs molécules qui sont liées au(x) groupe(s) d'ancrage,
**caractérisé en ce que**
les groupes d'ancrage sont sélectionnés identiques ou différents parmi le groupe des composés de formule générale (I)
(R²O)₂(O)P-L-(CH₂)ₓ-M-R¹ (1),
sachant que
R¹ représente le -COOH, -OH, -SH, -NH₂, du benzophénone ou des dérivés de benzophénone,
R¹ représente l'hydrogène, le -CH₂CH₃ ou -CH₃,
L représente une liaison simple ou -O,
M représente -(CH₂-CH₂-O)_{y},
x un nombre sélectionné parmi le groupe composé de 1 à 25 et
y un nombre sélectionné parmi le groupe composé de 1 à 25.

2. Stent selon la revendication 1, **caractérisé en ce que** la ou les biomolécules sont sélectionnées identiques ou différentes parmi le groupe composé des protéines, de préférence la protéine A, les enzymes, les facteurs de croissance, les anticorps, de préférence les anticorps CD 133 et les séquences de peptide, de préférence cycliques RGD.

3. Stent selon la revendication 1 ou 2, **caractérisé en ce que** le corps de stent est sélectionné parmi les métaux permanents ou dégradables ou les matériaux polymériques dégradables.

4. Procédé de fabrication d'un stent revêtu aux biomolécules suivant une des revendications 1 à 3, **caractérisé en ce que** le procédé comprend le fait que :
a) on se procure un ou plusieurs corps de base de stent,
b) le ou les corps de base de stent sont nettoyés,
c) on se procure un ou plusieurs groupes d'ancrage identiques ou différents sélectionnés parmi le groupe de composés de formule générale (I)
(R²O)₂(O)P-L-(CH₂)ₓ-M-R¹ (1),
sachant que
R¹ représente le -COOH, -OH, -SH, -NH₂, du benzophénone ou des dérivés de benzophénone,
R¹ représente l'hydrogène, le -CH₂CH₃ ou -CH₃,
L représente une liaison simple ou -O,
M représente -(CH₂-CH₂-O)_{y},
x un nombre sélectionné parmi le groupe composé de 1 à 25 et
y un nombre sélectionné parmi le groupe composé de 1 à 25,
d) le ou les corps de base de stent nettoyé(s) sont ensuite fonctionnalisés avec un ou plusieurs groupes d'ancrage identiques ou différents sélectionnés parmi le groupe de composés de formule générale (I) issus de c),
e) on se procure une ou plusieurs biomolécules identiques ou différentes du groupe composé de composés favorisant le dépôt de cellules endothéliales précurseurs à la surface du stent
et
f) une ou plusieurs biomolécules identiques ou différentes issues de e) sont liées au(x) corps de base fonctionnalisé(s) issus de d).

5. Procédé selon la revendication 4, **caractérisé en ce que**
g) le ou les stents suivant l'étape b) sont nettoyés dans un plasma d'oxygène et/ou par lavage au solvant, de préférence de la série de solvants dichlorométhane, acétone, méthanol et eau Millipore, et
h) sont le cas échéant séchés.

6. Procédé selon la revendication 4 ou 5, **caractérisé en ce que**
i) un ou plusieurs groupes d'ancrage sélectionnés parmi les composés de formule (I) suivant l'étape c), dans un ou plusieurs solvants sélectionnés parmi le groupe composé du méthanol, de l'éthanol, de l'acétone, du tétrahydrofurane, du diméthylfonuamide, du chloroforme, du diméthylsulfoxyde et du dichlorométhane, notamment
j) le ou des stents nettoyés suivant l'étape b) sont mis totalement ou partiellement en contact avec la solution des groupes d'ancrage des composés de formule (I),
k) le cas échéant, le ou les solvants sont évaporés totalement ou partiellement,
l) le cas échéant, le ou les stents sont mélangés thermiquement à un ou plusieurs groupes d'ancrage,
m) le cas échéant, sont ensuite lavés avec un ou plusieurs solvants et,
n) le cas échéant, sont ensuite séchés.

7. Procédé selon une des revendications 4 à 6, **caractérisé en ce que**
o) la ou les biomolécules suivant l'étape e) sont mises dans un ou plusieurs tampons exempts d'acide aminé,
p) le ou les stents fonctionnalisés sont mis totalement ou partiellement en contact avec la ou les biomolécules identiques ou différentes mises en solution,
q) sont le cas échéant ensuite lavés et
r) sont le cas échéant ensuite séchés.

8. Utilisation de composés de formule générale (I)
(R²O)₂(O)P-L-(CH₂)ₓ-M-R¹ (1),
sachant que
R¹ représente le -COOH, -OH, -SH, -NH₂, du benzophénone ou des dérivés de benzophénone,
R¹ représente l'hydrogène, le -CH₂CH₃ ou -CH₃,
L représente une liaison simple ou -O,
M représente -(CH₂-CH₂-O)_{y},
x un nombre sélectionné parmi le groupe composé de 1 à 25 et
y un nombre sélectionné parmi le groupe composé de 1 à 25
comme groupes d'ancrage pour biomolécules sur des corps de base de stent.

9. Utilisation de composés de formule générale (I)
(R²O)₂(O)P-L-(CH₂)ₓ-M-R¹ (1),
sachant que
R¹ représente le -COOH, -OH, -SH, -NH₂, du benzophénone ou des dérivés de benzophénone,
R¹ représente l'hydrogène, le -CH₂CH₃ ou -CH₃,
L représente une liaison simple ou -O,
M représente -(CH₂-CH₂-O)_{y},
x un nombre sélectionné parmi le groupe composé de 1 à 25 et
y un nombre sélectionné parmi le groupe composé de 1 à 25
comme groupes d'ancrage pour la fabrication d'un ou plusieurs stents suivant une des revendications 1 à 3.
